# EUROPEAN PATENT APPLICATION

(11) **EP 4 338 725 A1**
(43) Date of publication of application: **20.03.2024**
(21) Application number: 22807351.6
(22) Date of filing: 27.04.2022
(51) Int. Cl.: A61K 8/894, A61K 8/29, A61K 8/37, A61K 8/891, A61Q 1/02

(54) **WATER-BREAKING MAKEUP COSMETIC**

(30) Priority: 14.05.2021 JP 2021082320
(71) Applicant: SHIN-ETSU CHEMICAL CO., LTD., Tokyo 1000005 (JP)
(72) Inventor: KONISHI Masayuki, Tokyo 100-0005 (JP); AKABANE Emi, Annaka-shi, Gunma 379-0224 (JP)
(74) Representative: Mewburn Ellis LLP
(86) International application number: PCT/JP2022/018992
(87) International publication number: WO 2022/239649

(57) **Abstract**

This makeup cosmetic comprises:
(A) a crosslinking polyether modified silicone in which silicone chains are crosslinked with polyether chains: 0.1-4 mass%;
(B) a UV absorbing agent: 3.5-20 mass%;
(C) one or more types selected from cyclic or straight-chain dimethyl polysiloxanes having a kinetic viscosity of 4-100 mm²/s at 25°C: 1.9 mass% or less;
(D) an aqueous component: 40-90 mass%; and
(E) a powder: 1-25 mass%.
The makeup cosmetic exhibits lightweight feel, good feeling in use, and excellent water-breaking sensation and coating properties.

## Description

### TECHNICAL FIELD

The present invention relates to a water-breaking makeup cosmetic.

### BACKGROUND ART

Organic ultraviolet absorbers such as ethylhexyl methoxycinnamate are sometimes used in makeup cosmetics for the purpose of imparting an ultraviolet-protective effect. However, because these compounds have a poor compatibility with silicone, the stabilization of silicone-based cosmetics that are light to the touch is difficult. ON account of this, including a high level of organic ultraviolet absorber has only worsened the feel of the cosmetic.

Cosmetics that are water-in-oil emulsion type sunscreen products which have a light, fresh feel on use and are easy to use continuously and the texture of which does not worsen with the inclusion of an organic ultraviolet absorber have been developed in recent years as a measure for everyday protection against sunburn (Patent Document 1: JP-A 2013-91625). However, a drawback of water-in-oil type cosmetics is that they have a low water resistance and tend to come off upon exposure to sweat, water and the like.

At the same time, it is known that oil-in-water type cosmetics having a fresh feel on use can be obtained by using crosslinked polyether-modified silicones to produce emulsions which have a high water content and large droplets (Patent Document 2: JP-A 2001-2521). It is also known that water-breaking type cosmetics can be prepared by engineering the droplet size of such large-droplet emulsions.

Art that provides oil-in-water type water-breaking cosmetics formulated with an ultraviolet absorber is also known (Patent Document 3: JP-A 2011-219448, Patent Document 4: WO 2018/221174 A1, Patent Document 5: WO 2016/204947 A1). However, because research with nonvolatile dimethicones for the sake of stabilization has not been carried out, in cases where a crosslinked polyether-modified silicone is included, an ingredient such as an acrylic silicone is needed for stabilization, and including a high level of ultraviolet absorber has been difficult. Also, when stabilization is effected with, for example, a crosslinked polyether-modified silicone having alkyl branches instead of a crosslinked polyether-modified silicone, the cosmetic is heavier to the touch. When a high level of crosslinked polyether-modified silicone is included for the sake of stabilization, the viscosity rises and so the formulation becomes heavier to the touch, making it difficult to include powders such as coloring pigments that are essential to makeup cosmetics. In cases where a high level of dimethicone is included as a compatibilizing agent and the ultraviolet absorber concentration is lowered, the amount of oil phase increases; when pigments, etc. are added, the amount of the aqueous phase decreases, which can make it difficult to design a water-breaking type formulation. When an ultraviolet absorber is included, as mentioned above, a clean, light feel on use is wanted. Hence, there exists a desire for research on water-breaking makeup cosmetics which, rather than being stabilized according to the prior art, have been given a clean, light feel using a crosslinked polyether-modified silicone.

### PRIOR ART DOCUMENTS

### PATENT DOCUMENTS

Patent Document 1: JP-A 2013-91625
Patent Document 2: JP-A 2001-2521
Patent Document 3: JP-A 2011-219448
Patent Document 4: WO 2018/221174 A1
Patent Document 5: WO 2016/204947 A1

### SUMMARY OF INVENTION

### TECHNICAL PROBLEM

In light of the above circumstances, the object of the present invention is to provide a makeup cosmetic which is light to the touch and has a good feeling on use, a good water-breaking sensation and a good ease of application. In this invention, compositions for cosmetic use are sometimes referred to simply as "cosmetics."

### SOLUTION TO PROBLEM

The inventors have conducted intensive investigations aimed at achieving this object and have discovered as a result that the above issues can be resolved by, in a water-breaking makeup cosmetic containing (A) from 0.1 to 4 wt% of a crosslinked polyether-modified silicone having a silicone chain crosslinked with polyether chains, (B) from 3.5 to 20 wt% of an ultraviolet absorber, (D) from 40 to 90 wt% of an aqueous ingredient and (E) from 1 to 25 wt% of a powder, selecting (C) one or more cyclic or linear dimethylpolysiloxane having a kinematic viscosity at 25°C of from 4 to 100 mm²/s and setting the content thereof to 1.9 wt% or less. This discovery ultimately led to the present invention.

Accordingly, the invention provides the following water-breaking makeup cosmetic.
1. A water-breaking makeup cosmetic which includes:
   (A) from 0.1 to 4 wt% of a crosslinked polyether-modified silicone having a silicone chain crosslinked with polyether chains,
   (B) from 3.5 to 20 wt% of an ultraviolet absorber,
   (C) 1.9 wt% or less of one or more compound selected from cyclic or linear dimethylpolysiloxanes having a kinematic viscosity at 25°C of from 4 to 100 mm²/s,
   (D) from 40 to 90 wt% of an aqueous ingredient, and
   (E) from 1 to 25 wt% of a powder.
2. The water-breaking makeup cosmetic of 1 above, wherein the combined amount of components (B) and (E) is from 4.5 to 45 wt% of the overall cosmetic.
3. The water-breaking makeup cosmetic of 1 or 2 above, wherein component (A) is dimethicone/PEG-10/15 crosspolymer.
4. The water-breaking makeup cosmetic of any of 1 to 3 above which includes as component (D) from 38 to 85 wt% of water, based on the overall cosmetic.
5. The water-breaking makeup cosmetic of any of 1 to 4 above, further including (F) from 0.1 to 20 wt% of diphenylsiloxy phenyl trimethicone.
6. The water-breaking makeup cosmetic of any of 1 to 5 above, further including (G) from 0.1 to 20 wt% of ethyl methicone.
7. The water-breaking makeup cosmetic of any of 1 to 6 above, further including (H) from 0.1 to 1.0 wt% of a non-crosslinked silicone surfactant.

### ADVANTAGEOUS EFFECTS OF INVENTION

This invention makes it possible to provide water-breaking makeup cosmetics which are light to the touch and have a good feel on use, an excellent water-breaking sensation and an excellent ease of application (spread).

### DESCRIPTION OF EMBODIMENTS

The invention is described in detail below. Ingredient names in the invention are those cited in *Kesho̅hin Hydo̅ji Meisho̅* [Japanese Cosmetic Labeling Names] (KHM) or the International Nomenclature of Cosmetic Ingredients (INCI). In this Specification, when the name of an ingredient differs in these two lists, the KHM name is given first in lower case letters, followed by, in parenthesis, the capitalized INCI name. When the name from the two lists is the same, it is followed simply by "(INCI)."

### [Component (A)]

The crosslinked polyether-modified silicone having a silicone chain crosslinked with polyether chains (sometimes abbreviated below as "crosslinked polyether-modified silicone") serving as component (A) in the invention is a linear dimethylpolysiloxane that is crosslinked with a polyether. One specific example is the compound having the INCI name "Dimethicone/PEG-10/15 Crosspolymer." One crosslinked polyether-modified silicone may be used alone or two or more may be used in combination. In the cosmetic of the invention, component (A) is an ingredient which is essential for achieving both stabilization of the cosmetic and a water-breaking sensation.

The crosslinked polyether-modified silicone preferably includes and is swollen with its own weight or more of an oil that is liquid at 25°C. The oil that is liquid at 25°C is preferably an oil other than subsequently described component (C). Specific examples include phenyl silicones such as diphenylsiloxy phenyl trimethicone; silicones modified with an alkyl group of 2 to 18 carbon atoms, such as ethyl methicone and caprylyl methicone; hydrocarbon oils such as liquid paraffin, squalane, isododecane and isohexadecane; glyceride oils such as triethylhexanoin; ester oils such as isotridecyl isononanoate, N-acylglucamic acid esters, isodecyl neopentanoate and lauroyl sarcosinic acid esters; and natural animal and vegetable oils such as macadamia nut oil. Of these, diphenylsiloxy phenyl trimethicone is preferred. A specific example of the crosslinked polyether-modified silicone is KSG-270, from Shin-Etsu Chemical Co., Ltd.

The content of component (A) with respect to the overall cosmetic is from 0.1 to 4 wt%, preferably from 0.2 to 3 wt%, more preferably from 0.6 to 2 wt%, and even more preferably from 0.8 to 1.5 wt%.

### [Component (B)]

The ultraviolet absorber serving as component (B) in the invention is not particularly limited so long as it is an ingredient commonly used in cosmetics. One such ultraviolet absorber may be used alone or two or more may be used in combination. Examples of the ultraviolet absorber include Homosalate (INCI), Octocrylene (INCI), t-butyl methoxydibenzoylmethane (INCI: Butyl Methoxydibenzoylmethane), Ethylhexyl Salicylate (INCI), Diethylamino Hydroxybenzoyl Hexyl Benzoate (INCI), oxybenzone-6 (INCI: Benzophenone-6), oxybenzone-9 (INCI: Benzophenone-9), oxybenzone-1 (INCI: Benzophenone-1), Polysilicone-15 (INCI), octyl dimethoxybenzylidene dioxoimidazolidine propionate (INCI: Ethylhexyl Dimethoxybenzylidene Dioxoimidazolidine Propionate), oxybenzone-2 (INCI: Benzophenone-2), Terephthalylidene Dicamphor Sulfonic Acid (INCI), Ethylhexyl Triazone (INCI), methylbis(trimethylsiloxy)silyl isopentyl trimethoxycinnamate (INCI: Isopentyl Trimethoxycinnamate Trisiloxane), Drometrizole Trisiloxane (INCI), Ethylhexyl Dimethyl PABA (INCI), isopropyl p-methoxycinnamate (INCI: Isopropyl Methoxycinnamate), Ethylhexyl Methoxycinnamate (INCI), Bis-Ethylhexyloxyphenol Methoxyphenyl Triazine (INCI), oxybenzone-3 (INCI: Benzophenone-3), oybenzone-4 (INCI: Benzophenone-4), oxybenzone-5 (INCI: Benzophenone-5), Phenylbenzimidazole Sulfonic Acid (INCI), Methylene Bis-Benzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI) and Ethylhexyl Dimethoxybenzylidene Dioxoimidazolidine Propionate (INCI).

Also, a UVA absorber (e.g., Diethylamino Hydroxybenzoyl Hexyl Benzoate (INCI)) and a UVB absorber (e.g., Ethylhexyl Methoxycinnamate (INCI)) may be used together; each may also be freely combined.

Of these, from the standpoint of the ease of use, one or more organic ultraviolet absorber selected from ethylhexyl methoxycinnamate, diethylamino hydroxybenzoyl hexyl benzoate, ethylhexyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, homomenthyl salicylate and octocrylene is preferred.

The content of component (B) with respect to the overall cosmetic is from 3.5 to 20 wt%, preferably from 5 to 20 wt%, more preferably from 6 to 20 wt%, even more preferably from 7 to 12 wt%, and most preferably from 7.5 to 12 wt%.

### [Component (C)]

Component (C) of the invention is a cyclic or linear dimethylpolysiloxane having a kinematic viscosity at 25°C of from 4 to 100 mm²/s, preferably from 4 to 20 mm²/s. Examples of cyclic dimethylpolysiloxanes include Cyclopentasiloxane (INCI) and Cyclohexasiloxane (INCI). An example of a linear dimethyl polysiloxane is Dimethicone (INCI). It should be noted that component (C) is a dimethylpolysiloxane having four or more R²SiO_{2/2} groups. Even when component (C) is mixed with dimethicones of the formulas (R³SiO_{1/2})₂(R²SiO_{2/2})₂ and (R³SiO_{1/2})₂(R²SiO_{2/2})₃ having kinematic viscosities at 25°C of from 1.5 to 2 mm²/s to form a mixed oil having a kinematic viscosity of less than 4 mm²/s, the kinematic viscosity of component (C) is defined as the kinematic viscosity prior to mixture.

The kinematic viscosity in this invention indicates the measured value obtained with a Cannon-Fenske viscometer in accordance with JIS Z 8803:2011.

The content of component (C) with respect to the overall cosmetic is 1.9 wt% or less (from 0 to 1.9 wt%), preferably 1.0 wt% or less, more preferably less than 1.0 wt%, even more preferably 0.5 wt% or less, and most preferably 0 wt% (meaning that none is included). Component (C) is an optional ingredient and not an essential ingredient. Component (C) has a poor compatibility with component (B), and so affects the stability of the cosmetic. By setting the component (C) content below 1.9 wt%, the cosmetic can be stably emulsified even when the contents of component (B) and the subsequently described component (E) within the cosmetic are increased, resulting in a water-breaking makeup cosmetic which is light to the touch and has a good feel on use, a good water-breaking sensation and excellent ease of application (spread). When component (C) is included, the weight ratio of component (C) to component (B), expressed as (C)/(B), is preferably less than 0.5, more preferably less than 0.28, and even more preferably less than 0.15.

### [Component (D)]

Component (D) of the invention is an aqueous ingredient. The solubility in purified water at 25°C is 0.01 g/100 g or more. Examples include water, moisturizers, lower alcohols, water-soluble macromolecular compounds, skin-beautifying ingredients, water-soluble inorganic salts (pH adjustors, etc.), and water-soluble preservatives. These may be used singly or two or more may be used in suitable combination.

Examples of water include purified water or fruit or plant distilled water commonly used in cosmetics, and also the following: Sea Water (INCI), hot-spring water (Onsen-Sui) and Peat Water (INCI).

Examples of moisturizers include sugar alcohols such as Sorbitol (INCI), Maltose (INCI) and Xylitol (INCI); polyhydric alcohols such as BG (INCI: Butylene Glycol), PG (INCI: Propylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI) and polyethylene glycol; and also Glucose (INCI), Glyceryl Glucoside (INCI), Betaine (INCI), Sodium Chondroitin Sulfate (INCI), PCA-Na (INCI: Sodium PCA), Methyl Gluceth-10 (INCI) and Methyl Gluceth-20 (INCI). The moisturizer content with respect to the overall cosmetic is preferably from 2 to 52 wt%, more preferably from 5 to 13 wt%, and even more preferably from 7 to 10 wt%. By including a moisturizer, it is possible to increase the emulsification stability and adjust the water-breaking sensation of the cosmetic.

Examples of lower alcohols (5 carbons or less) include Ethanol (INCI) and Isopropanol (INCI).

Examples of water-soluble macromolecular compounds include natural water-soluble macromolecular compounds such as Carrageenan (INCI), Sodium Hyaluronate (INCI) and Xanthan Gum (INCI); semisynthetic water-soluble macromolecular compounds such as Hydroxyethyl Cellulose (INCI), Hydroxypropyl Methylcellulose (INCI) and Carboxymethyl Cellulose (INCI); synthetic water-soluble polymeric compounds such as Polyvinyl Alcohol (INCI) and Carbomer (INCI); and inorganic water-soluble macromolecular compounds such as Bentonite (INCI) and Laponite (INCI).

Examples of skin-beautifying ingredients include whitening agents such as Arbutin (INCI), ascorbic acid and derivatives thereof; anti-inflammatory agents such as Allantoin (INCI) and glycyrrhizates; and blood circulation promoters such as benzyl nicotinate.

Examples of water-soluble inorganic salts include water-soluble inorganic salts such as Sodium Chloride (INCI), Magnesium Sulfate (INCI) and Sodium Citrate (INCI), and pH adjustors.

Examples of water-soluble preservatives include Methylparaben (INCI), Phenoxyethanol (INCI), Imidazolidinyl Urea (INCI), Ethylhexylglycerin (INCI), Glyceryl Caprylate (INCI), Caprylhydroxamic Acid (INCI) and Polyaminopropyl Biguanide (INCI).

The content of component (D) with respect to the overall cosmetic is from 40 to 90 wt%, preferably from 50 to 85 wt%, more preferably from 60 to 80 wt%, and even more preferably from 60 to 70 wt%. By setting the component (D) content to 40 wt% or more, the water-breaking sensation can be increased; by setting this to not more than 90 wt%, the stability is further increased. The water content with respect to the overall cosmetic is preferably from 38 to 85 wt%, more preferably from 45 to 80 wt%, even more preferably from 50 to 70 wt%, and still more preferably from 50 to 60 wt%. By setting the amount of water to 38 wt% or more, a sticky feeling can be suppressed; by setting the amount to not more than 85 wt%, the stability can be further increased.

### [Powder (E)]

Component (E) of the invention is a powder. The powder is exemplified by finely divided metal oxide powders, hydrophobized coloring pigments, inorganic powders, metal powders, organic powders and inorganic/organic composite powders. These may be used singly or two or more may be used in suitable combination. It should be noted that component (E) excludes ingredients corresponding to above component (B).

### • Finely Divided Metal Oxide Powders

Finely divided metal oxides that may be used in this invention include one or more selected from finely divided titanium oxide (INCI: Titanium Dioxide), iron-containing titanium oxide, Zinc Oxide (INCI), Cerium Oxide (INCI), and composites thereof. These metal oxides may be composite powders with other powders. The average primary particle size is preferably 200 nm or less, and more preferably 120 nm or less. At a particle size larger than this, the ultraviolet protective function may decrease and a white residue may form. The average primary particle size can be measured by way of, for example, transmission electron micrographs.

The finely divided metal oxide is not particularly limited, and may be untreated or may have a known surface treatment commonly used in cosmetics. Examples of inorganic treatments include Silica (INCI) coating, Alumina (INCI) coating and Aluminum Hydroxide (INCI) coating. Examples of organic treatments include silanes and silylating agents such as Triethoxycaprylylsilane (INCI), silicone oils such as Dimethicone (INCI), Hydrogen Dimethicone (INCI) and Triethoxysilylethyl Polydimethylsiloxyethyl Hexyl Dimethicone (INCI), waxes, paraffins, organofluorine compounds such as perfluoroalkyl phosphates, surfactants, amino acids such as N-acylglutamic acid, and metal soaps such as Aluminum Stearate (INCI) and Magnesium Myristate (INCI). In particular, Triethoxysilylethyl Polydimethylsiloxyethyl Hexyl Dimethicone (INCI) exhibits a high dispersibility with respect to both silicones and oils, and so is applied to sunscreens and foundations. Triethoxycaprylylsilane (INCI) and metal soap treatment are preferred in terms of the compatibility with component (B) and subsequently described component (D). These surface treatments may be used singly or two or more may be combined according to the intended purpose.

Commercial products may also be used as these surface-treated finely divided metal oxides. For example, finely divided titanium oxides are commercially available under trade names such as STR-100C-LP, STR-100A-LP, STR-100W, STR-100W-LP, STR-100C-LF and STR-40-LP (Sakai Chemical Co., Ltd.), MT-01, MT-05, MT-100Z, MT-100TV, MT-100AQ, MT-150EX, MT-500B, MT-505SAS, MT-700B, MT-014Z and SMT-500SAS (Tayka Corporation), and ST-455, ST-455WS, ST-457ECS and ST-495M (Chitan Kogyo Co., Ltd.). Finely divided zinc oxides are commercially available under trade names such as FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT and FINEX-30S-LPT (Sakai Chemical Co., Ltd.), and MZ-150, MZ-200, MZ-300, MZ-306X, MZ-500HP, MZ-505T, MZ-506X, MZY-203S, MZY-210M3S, TMZ-HA1 and MZX-5080TS (Tayca Corporation).

### • Hydrophobized Coloring Pigments

The coloring pigment of the hydrophobized coloring pigment is not particularly limited, provided that it is a pigment normally used for the purpose of coloring cosmetics. Examples include red iron oxide (INCI: Iron Oxides), yellow iron oxide (INCI: Iron Oxides), white titanium oxide (INCI: Titanium Dioxide), black iron oxide (INCI: Iron Oxides), Ultramarines (INCI), Prussian blue (INCI: Ferric Ferrocyanide, Ferric Ammonium Ferrocyanide), Manganese Violet (INCI), cobalt titanate (INCI: Cobalt Titanium Oxide), chromium hydroxide (INCI: Chromium Hydroxide Green), chromium oxide (INCI: Chromium Oxide Greens), Cobalt Aluminum Oxide (INCI), titanium/titanium oxide sinter (INCI: Titanium/Titanium Dioxide), Lithium Cobalt Titanate (INCI), Iron Oxide/Titanium Dioxide Sinter (INCI), composites doped with differing metals such as iron oxide-doped titanium oxide (INCI: Iron Oxides, Titanium Dioxide), Titanium Nitride (INCI), ferrous hydroxide (INCI: Iron Hydroxide), inorganic brown pigments such as gamma iron oxide, inorganic yellow pigments such as yellow ochre, and organic pigments such as lakes of tar dyes and lakes of natural dyes. Any of these may be used.

The hydrophobized coloring pigment may have any of various particle shapes, such as spherical, approximately spherical, rod-like, spindle-like, petal-like, reed-like or amorphous shapes. So long as it is able to impart color to the cosmetic, the geometric form of the pigment is not particularly limited. From the standpoint of the hiding power, a pigment having a particle size, meaning here the volume mean particle size (MV value), in the range of 150 to 600 nm is preferred. The volume mean particle size can be measured with a transmission electron microscope (TEM) or the like. At less than 150 nm, the hiding power is low and so the cosmetic coloring efficiency may be low. At a volume mean particle size larger than 600 nm, the feel on use of the cosmetic may worsen.

In addition, the pigment according to the invention may be partially or entirely surface-treated with an inorganic compound such as Alumina (INCI), Aluminum Hydroxide (INCI), Silica (INCI) or Hydrated Silica (INCI).

Here, "hydrophobization" refers to surface treatment of the above-described coloring pigment with a hydrophobizing agent The surface hydrophobizing agent for the coloring pigment of the invention is not particularly limited, so long as it can impart hydrophobicity. Exemplary treatment agents include silicone treatment agents, waxes, paraffins, organofluorine compounds such as perfluoroalkyl phosphates, surfactants, amino acids such as N-acylglutamic acids, and metal soaps such as Aluminum Stearate (INCI) and Magnesium Myristate (INCI).

Of these, silicone treatment agents can be preferably used. Examples include silanes and silylating agents such as Triethoxycaprylylsilane (INCI) and Trimethoxysilyl Dimethicone (INCI), silicone oils such as Dimethicone (INCI), Hydrogen Dimethicone (INCI) and Triethoxysilylethyl Polydimethylsiloxyethyl Hexyl Dimethicone (INCI), and silicone compounds such as Acrylates/Tridecyl Acrylate/Triethoxysilylpropyl Methacrylate/Dimethicone Methacrylate Copolymer (INCI) and Acrylates/Dimethicone Copolymer (INCI). The silicone powder treatment agents mentioned in JP 3912961 can be preferably used as the silicone treatment agent. In particular, even when the dispersing medium that disperses the highly hydrophobized coloring pigment is a mixture composed of a silicone and a hydrocarbon, Triethoxysilylethyl Polydimethylsiloxyethyl Hexyl Dimethicone (INCI), which is a dimethylpolysiloxane having pendent triethoxysilyl groups, polydimethylsiloxyethyl groups and hexyl groups, can be effectively used as the surface hydrophobizing agent because it exhibits a high affinity. A single surface hydrophobizing agent may be used alone or two or more may be used in combination.

Examples of commercially available coloring pigments that have been subjected to hydrophobizing surface treatment include the KTP-09 Series, especially KTP-09W, KTP-09R, KTP-09Y and KTP-09B (from Shin-Etsu Chemical Co., Ltd.).

### • Inorganic Powders

Exemplary inorganic powders include fine particles made of, for example, Zirconium Dioxide (INCI), Zinc Oxide (INCI), Cerium Oxide (INCI), Magnesium Oxide (INCI), Barium Sulfate (INCI), calcium sulfate (INCI: Calcium Sulfate), Magnesium Sulfate (INCI), Calcium Carbonate (INCI), Magnesium Carbonate (INCI), Talc (INCI), cleaved talc (INCI: Talc), Mica (INCI), Kaolin (INCI), sericite (INCI: Mica), Synthetic Fluorphlogopite (INCI), Biotite (INCI), Potassium Silicate (INCI), Silica (INCI), fumed silica, Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI), Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), Zeolite (INCI), Alumina (INCI), Aluminum Hydroxide (INCI), Boron Nitride (INCI) or Glass (INCI). Examples of inorganic pearlescent coloring pigments include pearlescent pigments such as titanium oxide-coated mica, Bismuth Oxychloride (INCI), Titanium Oxide (INCI)-coated Bismuth Oxychloride (INCI), Titanium Oxide (INCI)-coated Talc (INCI), natural pearl essence and Titanium Oxide (INCI)-coated colored mica. These pearlescent pigments are not particularly limited, and may be untreated or may have a known surface treatment commonly used in cosmetic products.

### · Metal Powders

Examples of metal powders include metal fine particles, such as Aluminum Powder (INCI), Copper Powder (INCI) and Silver Powder (INCI).

### · Organic Powders

Examples of organic powders include powders made of silicones, polyamides, polyacrylic acid/polyacrylic acid esters, polyesters, polyethylene, polypropylene, polystyrene, styrene/acrylic acid copolymers, divinylbenzene/styrene copolymers, polyurethanes, vinyl resins, urea resins, melamine resins, benzoguanamine, polymethyl benzoguanamine, tetrafluoroethylene, polymethyl methacrylate, cellulose, silk, nylon, phenolic resins, epoxy resins and polycarbonates. In particular, exemplary silicones include silicone resin particles (e.g., Polymethylsilsesquioxane (INCI), commercially available as KMP-590, KMP-591, KMP-592, etc. from Shin-Etsu Chemical Co., Ltd.), silicone rubber powders (commercially available as KMP-597, KMP-598, etc. from Shin-Etsu Chemical Co., Ltd.), and silicone resin-coated silicone rubber powders (e.g., Vinyl Dimethicone/Methicone Silsesquioxane Crosspolymer (INCI), commercially available as KSP-100, KSP-101, KSP-102 and KSP-105 from Shin-Etsu Chemical Co., Ltd.; Diphenyl Dimethicone/Vinyl Diphenyl Dimethicone/Silsesquioxane Crosspolymer (INCI), commercially available as KSP-300 from Shin-Etsu Chemical Co., Ltd.; Polysilicone-1 Crosspolymer (INCI), commercially available as KSP-411 from Shin-Etsu Chemical Co., Ltd.; and Polysilicone-22 (INCI), commercially available as KSP-441 from Shin-Etsu Chemical Co., Ltd.). These may be dispersed beforehand in water or oil (specific examples include KM-9729, KM-440 and KG-016, from Shin-Etsu Chemical Co., Ltd.). Additional examples of organic powders include metal soaps such as Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI) and Potassium Cetyl Phosphate (INCI). Further examples of organic powders include organic dyes, such as the tar dyes Red 3, Red 104(1) (INCI: Red 28, Red 28 Lake), Red 106, Red 201 (INCI: Red 6), Red 202 (INCI: Red 7), Red 204, Red 205, Red 220 (INCI: Red 34), Red 226 (INCI: Red 30), Red 227 (INCI: Red 33, Red 33 Lake), Red 228 (INCI: Red 36), Red 230(1) (INCI: Red 22, Red 33 Lake), Red 230(2), Red 401, Red 505, Yellow 4 (INCI: Yellow 5), Yellow 5 (INCI: Yellow 6, Yellow 6 Lake), Yellow 202(1) (INCI: Yellow 8), Yellow 203 (INCI: Yellow 10, Yellow 10 Lake), Yellow 204 (INCI: Yellow 11), Yellow 401, Blue 1 (INCI: Blue 1, Blue 1 Lake), Blue 2, Blue 201, Blue 205 (INCI: Blue 4), Blue 404, Green 3 (INCI: Green 3, Green 3 Lake), Green 201 (INCI: Green 5), Green 202 (INCI: Green 6), Green 204 (INCI: Green 8), Green 205, Orange 201 (INCI: Orange 5), Orange 203 (INCI: Pigment Orange 5), Orange 204, Orange 205 (INCI: Orange 4, Orange 4 Lake), Orange 206 (INCI: Orange 10) and Orange No. 207 (INCI: Orange 11); and natural dyes such as Cochineal (INCI), Laccaic Acid (INCI), Carthamus Tinctorius (Safflower) Flower Extract (INCI), Lithospermum Officinale Root Extract (INCI), gardenia yellow and gardenia blue (INCI: Hydrolyzed Gardenia Florida Extract).

### · Inorganic/Organic Composite Powders

Examples of inorganic/organic composite powders include composite powders in which the surfaces of inorganic powder particles are coated with an organic powder by a commonly known and used method.

Of the above, finely divided metal oxide powders and hydrophobized coloring pigments are especially preferred. The content of finely divided metal oxide powder with respect to the overall cosmetic is preferably from 1 to 25 wt%, more preferably from 3 to 15 wt%, and even more preferably from 6 to 10 wt%. At a content below 1 wt%, a sufficient ultraviolet-blocking effect may not be obtained. The content of hydrophobized coloring pigment with respect to the overall cosmetic is preferably from 1 to 25 wt%, more preferably from 3 to 15 wt%, and even more preferably from 6 to 10 wt%. At a content below 1 wt%, sufficient blocking effects and coloring effects may not be obtained.

The content of component (E) with respect to the overall cosmetic is from 1 to 25 wt%, preferably from 3.0 to 20 wt%, more preferably from 6.0 to 20 wt%, and even more preferably from 12 to 20 wt%. At a content greater than 25 wt%, the formulation may be difficult to stabilize.

By using component (B) and component (E) together, an ultraviolet protective effect and an opacifying effect can both be achieved. In addition, when the combined amount of components (B) and (E) is large, a higher ultraviolet protective effect is obtained, but designing the formulation is difficult. A water-breaking cosmetic that has a good feel can be obtained even when higher amounts of components (B) and (E) are included in accordance with this invention. The combined amount of components (B) and (E) with respect to the overall cosmetic is preferably from 4.5 to 45 wt%, more preferably from 6.0 to 30 wt%, even more preferably from 12 to 30 wt%, and still more preferably from 19 to 30 wt%.

### [Component (F)]

Component (F) of the invention is Diphenylsiloxy Phenyl Trimethicone (INCI). This is commercially available as, for example, KF-56A from Shin-Etsu Chemical Co., Ltd.

When component (F) is included, the content with respect to the overall cosmetic is preferably from 0.1 to 20 wt%, more preferably from 2.0 to 15 wt%, even more preferably from 3 to 12 wt%, and still more preferably from 4 to 10 wt%. Component (F) is desirable because it has a very good compatibility with component (B) and also makes the cosmetic light to the touch.

### [Component (G)]

Component (G) is Ethyl Methicone (INCI). It is commercially available as, for example, KF-4422 from Shin-Etsu Chemical Co., Ltd.

When component (G) is included, the content with respect to the overall cosmetic is preferably from 0.1 to 20 wt%, more preferably from 2.0 to 15 wt%, even more preferably from 3.0 to 12 wt%, and still more preferably from 4.0 to 10 wt%. Component (G) is desirable because it has a good compatibility with component (B) and also makes the cosmetic light to the touch.

### [Component (H)]

Component (H) of the invention is a non-crosslinked silicone surfactant in which some of the methyl groups on the linear or branched silicone main chain are substituted with hydrophilic groups such as polyethylene glycol or polyglycerol. Unlike component (A), this is a silicone surfactant in which the main chains are not crosslinked with hydrophilic groups. Preferred examples include linear or branched polyoxyethylene-modified organopolysiloxanes, linear or branched polyoxyethylenepolyoxypropylene-modified organopolysiloxanes, linear or branched polyoxyethylene/alkyl-co-modified organopolysiloxanes, linear or branched polyoxyethylenepolyoxypropylene/alkyl-co-modified organopolysiloxanes, linear or branched polyglycerol-modified organopolysiloxanes, linear or branched polyglycerol/alkyl-co-modified organopolysiloxanes and linear or branched pyrrolidone-modified organopolysiloxanes. In these surfactants, the content of the hydrophilic polyoxyethylene groups, polyoxyethylenepolyoxypropylene groups or polyglycerol residues preferably accounts for 10 to 70 wt% of the molecule. Commercially available examples include KF-6011, KF-6013, KF-6043, KF-6017, KF-6028, KF-6038, KF-6048, KF-6100, KF-6104, KF-6105, KF-6106 and KF-6115 from Shin-Etsu Chemical Co., Ltd. These may be used singly or two or more may be used in suitable combination.

For example, in a combination of a linear or branched polyoxyethylene-modified organopolysiloxane and a linear or branched polyglycerol-modified organopolysiloxane, it is possible to further adjust the water-breaking sensation and the stability of the cosmetic. When a linear or branched polyoxyethylene-modified organopolysiloxane is included, the content thereof with respect to the overall cosmetic is preferably from 0.05 to 0.5 wt%, and more preferably from 0.2 to 0.3 wt%. A polyoxyethylene-modified organopolysiloxane content in excess of 0.5 wt% may affect the water-breaking sensation; at a content below 0.05 wt%, the cosmetic may become unstable. When a linear or branched polyglycerol-modified organopolysiloxane is included, the content with respect to the overall cosmetic is preferably from 0.05 to 1.0 wt%, and more preferably from 0.1 to 0.6 wt%.

More specifically, Lauryl PEG-9 Polydimethylsiloxyethyl Dimethicone (INCI) and Lauryl Polyglyceryl-3-Polydimethylsiloxyethyl Dimethicone (INCI) are preferable from the standpoint of compatibility with component (B); Lauryl Polyglyceryl-3-Polydimethylsiloxyethyl Dimethicone (INCI) is especially preferable from the standpoint of the water-breaking sensation.

When component (H) is included, the content thereof with respect to the overall cosmetic is preferably from 0.1 to 1.0 wt%, more preferably from 0.1 to 0.8 wt%, even more preferably from 0.2 to 0.8 wt%, and still more preferably from 0.5 to 0.8 wt%.

The cosmetic of the invention may include also optional ingredients other than the above ingredients in suitable amounts within ranges that do not detract from the advantageous effects of the invention. Such other optional ingredients are exemplified by: (1) oily ingredients other than components (B), (F) and (G), (2) film-forming agents, (3) surfactants other than component (H), (4) compositions of a crosslinked organopolysiloxane other than component (A) and an oil that is liquid at 25°C, and (5) other additives. One of these may be used alone or two or more may be used in combination.

### (1) Oily Ingredients other than Components (B), (F) and (G)

Examples of oily ingredients other than components (B), (F) and (G) include silicone oils, hydrocarbon oils, fluorocarbon oils, ester oils, natural animal and vegetable oils and semisynthetic oils.

The silicone oils refer to dimethylpolysiloxanes other than component (C) and modified silicone oils. Specific examples include alkyl-modified silicones such as Dimethicone (INCI) having a kinematic viscosity at 25°C of 1.5 to 2 mm²/s, Trisiloxane (INCI), Methyl Trimethicone (INCI), Ethyl Trisiloxane (INCI) and Hexyl Dimethicone (INCI); long-chain alkyl-modified silicones such as Caprylyl Methicone (INCI); linear or branched organopolysiloxanes such as Phenyl Trimethicone (INCI), Diphenyl Dimethicone (INCI) and Tetraphenyl Dimethyl Disiloxane (INCI); aromatic group-modified silicones such as tetramethyltetraphenylcyclotetrasiloxane and other cyclic organopolysiloxanes; amino-modified organopolysiloxanes such as Amodimethicone (INCI) and Aminopropyl Dimethicone (INCI); pyrrolidone-modified organopolysiloxanes such as PCA Dimethicone (INCI); pyrrolidone carboxylic acid-modified organopolysiloxanes, amino acid-modified silicones and fluorine-modified silicones.

The hydrocarbon oils are exemplified by acyclic and cyclic hydrocarbon oils. Specific examples include Olefin Oligomers (INCI), isoparaffins such as C13-14 Isoalkane (INCI), Isododecane (INCI), Undecane (INCI), Dodecane (INCI), Isohexadecane (INCI), Hydrogenated Polyisobutene (INCI), Squalane (INCI), Mineral Oil (INCI), Coconut Alkanes (INCI) and C13-15 Alkane (INCI).

Examples of the fluorocarbon oils include perfluoropolyether, perfluorodecalin and perfluorooctane.

Examples of the esters include Diisobutyl Adipate (INCI), dihexyldecyl adipate, Diheptylundecyl Adipate (INCI), n-alkyl glycol monoisostearates such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), cetyl octanoate (INCI: Cetyl Ethylhexanoate), octyl dodecyl esters such as Octyldodecyl Stearoyl Stearate (INCI), Oleyl Oleate (INCI), Octyldodecyl Oleate (INCI), Decyl Oleate (INCI), neopentyl glycol dioctanoate (INCI: Neopentyl Glycol Diethylhexanoate), Neopentyl Glycol Dicaprate (INCI), Diisostearyl Malate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitic acid esters such as Isopropyl Palmitate (INCI), Ethylhexyl Isopalmitate (INCI) and hexyldecyl palmitate (INCI: Isocetyl Palmitate, Hexyldecyl Palmitate), Cholesteryl Hydroxystearate (INCI), myristic acid esters such as Isopropyl Myristate (INCI), Octyldodecyl Myristate (INCI) and Myristyl Myristate (INCI), Ethylhexyl Laurate (INCI), Hexyl Laurate (INCI), Dioctyldodecyl Lauroyl Glutamate (INCI) and Isopropyl Lauroyl Sarcosinate (INCI).

Examples of the ester oils include glyceride oils such as Triethylhexanoin (INCI), Caprylic/Capric Triglyceride (INCI), Cocoglycerides (INCI), Caprylic/Capric/Succinic Triglyceride (INCI) and Caprylic/Capric Glycerides (INCI).

Examples of the natural animal and vegetable oils and the semisynthetic oils include natural vegetable oils such as avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), perilla oil, olive oil (INCI: Olea Europaea (Olive) Fruit Oil), nutmeg oil (INCI: Torreya Californica (California Nutmeg) Oil), citronella oil (INCI: Cymbopogon Nardus (Citronella) Oil), Torreya Nucifera Seed Oil (INCI), Kyounin Yu (INCI), wheatgerm oil (INCI: Triticum Vulgare (Wheat) Germ Oil), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), Camellia Kissi Seed Oil (INCI), safflower oil (INCI: Carthamus Tinctorius (Safflower) Seed Oil), soybean oil (INCI: Glycine Soja (Soybean) Oil), Camellia Sinensis Seed Oil (INCI), Camellia Japonica Seed Oil (INCI), evening primrose oil (INCI: Oenothera Biennis (Evening Primrose) Oil), rapeseed oil (INCI: Rape Shushi Yu), germ oils such as corn germ oil (INCI: Zea Mays (Corn) Germ Oil), apricot kernel oil, palm oil (INCI: Elaeis Guineensis (Palm) Oil), palm kernel oil (INCI: Elaeis Guineensis (Palm) Kernel Oil), castor seed oil (INCI: Ricinus Communis (Castor) Seed Oil), sunflower seed oil (INCI: Helianthus Annuus (Sunflower) Seed Oil), grapeseed oil (INCI: Vitis Vinifera (Grape) Seed Oil), jojoba seed oil (INCI: Simmondsia Chinensis (Jojoba) Seed Oil), macadamia seed oil (INCI: Macadamia Ternifolia Seed Oil), Limnanthes Alba (Meadowfoam) Seed Oil (INCI), cottonseed oil (INCI: Gossypium Herbaceum (Cotton) Seed Oil), coconut oil (INCI: Cocos Nucifera (Coconut) Oil) and peanut oil; natural animal oils such as Shark Liver Oil (INCI), Cod Liver Oil (INCI), Fish Liver Oil (INCI), Turtle Oil (INCI), Mink Oil (INCI) and Egg Oil (INCI); and semisynthetic oils and fats such as Hydrogenated Coconut Oil (INCI) and Lanolin Oil (INCI).

In above Ingredient (1), silicone oils, hydrocarbon oils and ester oils are preferred. Dimethicone (INCI) having a kinematic viscosity of 1.5 to 2 mm²/s, Trisiloxane (INCI) and Methyl Trimethicone (INCI) are preferred as the silicone oils because a very light feeling on use can be obtained. When included, the content thereof with respect to the overall cosmetic is preferably from 0.1 to 10 wt%, more preferably from 1 to 8 wt%, and even more preferably from 2 to 8 wt%. Examples of commercially available products include KF-96A-1cs, KF-96L-1.5cs, KF-96L-2cs and TMF-1.5 from Shin-Etsu Chemical Co., Ltd.

Isododecane (INCI), Undecane (INCI), Dodecane (INCI), Isohexadecane (INCI) and C13-15 Alkane (INCI) are especially preferred as the hydrocarbon oils.

Triethylhexanoin (INCI), Isononyl Isononanoate (INCI), Isotridecyl Isononanoate (INCI), Cetyl Ethylhexanoate (INCI), Caprylic/Capric Triglyceride (INCI), Ethylhexyl Isopalmitate (INCI) and Hexyl Laurate (INCI) are especially preferred as the ester oils.

### (2) Film-Forming Agents

The film-forming agent is not particularly limited, provided that it is a material which can normally be included in cosmetics. Specifically, latexes of, for example, polyvinyl alcohol, polyvinylpyrrolidone, polyvinyl acetate and alkyl polyacrylates, dextrin, cellulose derivatives such as alkyl celluloses and nitrocellulose, siliconized polysaccharide compounds such as tri(trimethylsiloxy)silylpropylcarbamic acid pullulan, acrylic-silicone graft copolymers such as (alkyl acrylate/dimethicone) copolymers, silicone resins such as Trimethylsiloxysilicate (INCI), silicone-modified polynorbornene, silicone-based resins such as fluorine-modified silicone resins, fluorocarbon resins, aromatic hydrocarbon resins, polymer emulsion resins, terpene resins, polybutene, polyisoprene, alkyd resins, polyvinylpyrrolidone-modified polymers, rosin-modified resins and polyurethanes may be used.

Of these, silicone-type film-forming agents are especially preferred. Silicone-type film-forming agents that may be used include, but are not limited to: tri(trimethylsiloxy)silylpropylcarbamic acid pullulan (commercially available, as a product dissolved in a solvent, from Shin-Etsu Chemical Co., Ltd. as TSPL-30-D5, ID), (alkyl acrylate/dimethicone) copolymer (commercially available, as a product dissolved in a solvent, from Shin-Etsu Chemical Co., Ltd. as KP-543, KP-545, KP-549, KP-550, KP-545L, etc.), Trimethylsiloxysilicate (INCI) (commercially available, as a product dissolved in a solvent, from Shin-Etsu Chemical Co., Ltd. as KF-7312J, X-21-5250, etc.), silicone-modified polynorbornene (commercially available, as a product dissolved in a solvent, from Shin-Etsu Chemical Co., Ltd. as NBN-30-ID, etc.) and organosiloxane-grafted polyvinyl alcohol polymers. One, two or more film-forming agent may be used. When this ingredient is included, the content thereof with respect to the overall cosmetic is preferably from 0.1 to 20 wt%.

### (3) Surfactants Other than (H)

The surfactant is not particularly limited. There are nonionic, anionic, cationic and amphoteric surfactants. Any surfactant that is commonly used in cosmetics may be used. In cases where a crosslinked organopolysiloxane such as a partially crosslinked polyether-modified silicone or a partially crosslinked polyglycerol-modified silicone is used, in a composition made up of this crosslinked organopolysiloxane and an oil that is liquid at room temperature, it is preferable for the crosslinked organopolysiloxane to contain at least its own weight of the liquid oil and to swell with respect to the liquid oil.

Specific examples of crosslinked organopolysiloxanes that contain and are swollen with a liquid oil include KSG-310, KSG-320, KSG-330, KSG-340, KSG-320Z, KSG-350Z, KSG-710, KSG-810, KSG-820, KSG-830, KSG-840, KSG-820Z and KSG-850Z from Shin-Etsu Chemical Co., Ltd.

### (4) Compositions of Crosslinked Organopolysiloxane Other than Component (A) and an Oil that is Liquid at 25°C

A crosslinked organopolysiloxane may be included in this invention. This crosslinked organopolysiloxane is not particularly limited, so long as it is one that is normally used in cosmetics. One such compound may be used alone or two or more may be used in suitable combination. This crosslinked organopolysiloxane, unlike the above powders, does not have a spherical shape. Also, unlike the above surfactants, it is a compound which lacks polyether or polyglycerol moieties in the molecular structure. On account of swelling with component (1), it is an elastomer having structural viscosity.

Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI) and Lauryl Polydimethylsiloxyethyl Dimethicone/Bis-Vinyl Dimethicone Crosspolymer (INCI). These are commercially available in a swollen form containing an oil that is liquid at room temperature. Specific examples include KSG-15, KSG-1510, KSG-16, KSG-1610, KSG-18A, KSG-19, KSG-41A, KSG-42A, KSG-43, KSG-44, KSG-042Z, KSG-045Z and KSG-048Z. Of these, KSG-18A, swollen with diphenylsiloxy phenyl trimethicone (above component (F)), is especially preferred. When this ingredient is included, the content thereof, in terms of solids with respect to the overall cosmetic, is preferably from 0.01 to 30 wt%.

### (5) Other Additives

Other additives include oil-soluble gelling agents, water-soluble thickeners, antiperspirants, preservatives/bactericides, fragrances, antioxidants, chelating agents, algefacients, anti-inflammatory agents, skin beautifying ingredients (whitening agents, cell activators, skin roughness improvers, circulation promoting ingredients, skin astringents, antiseborrheic agents, etc.), vitamins, amino acids, nucleic acids, hormones and inclusion compounds. It should be noted that, even if mentioned below, substances corresponding to component (D) or component (E) are excluded from such "other additives."

### · Oil-Soluble Gelling Agents

Examples of oil-soluble gelling agents include metal soaps, such as aluminum stearate, magnesium stearate and zinc myristate; amino acid derivatives, such as N-lauroyl-L-glutamic acid and α,γ-di-n-butylamine; dextrin fatty acid esters, such as dextrin palmitate, dextrin stearate and dextrin 2-ethylhexanoate palmitate; sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; disteardimonium hectorite, stearalkonium hectorite, organic-modified clay minerals such as hectorite, esters that are pastes at 25°C, white petrolatum, lanolin, solid waxes and silicone waxes. The oil-soluble gelling agent content is preferably from 0 to 12 wt%, and more preferably from 0 to 8 wt%. Because the water-breaking cosmetic of the invention can be prepared as a stable emulsion without including an oil-soluble gelling agent, it is preferable to not include such an agent.

### · Antiperspirants

Examples of antiperspirants include aluminum hydroxyhalides such as aluminum chlorohydrate and aluminum chlorohydroxy allantoinate, aluminum halides such as aluminum chloride, aluminum allantoinate, tannic acid, persimmon tannin, aluminum potassium sulfate, zinc oxide, zinc p-phenolsulfonate, burnt alum, aluminum zirconium tetrachlorohydrate and aluminum zirconium trichlorohydrex glycine. In particular, aluminum hydroxyhalides, aluminum halides, and complexes or mixtures of these with zirconyl oxyhalides and zirconyl hydroxyhalides (e.g., aluminum zirconium tetrachlorohydrate and aluminum zirconium trichlorohydrex glycine) are preferred as ingredients which exhibit highly advantageous effects.

### · Preservatives and Bactericides

Examples of preservatives and bactericides include benzoic acid, sorbic acid, salicylic acid, isopropyl methyl phenol, carbolic acid, p-chloro-m-cresol, hexachlorophene, benzalkonium chloride, chlorhexidine chloride, trichlorocarbanilide, iodopropynyl butylcarbamate, polylysine, photosensitizers, silver and plant extracts.

### · Fragrances

Exemplary fragrances include natural fragrances and synthetic fragrances. Examples of natural fragrances include fragrances isolated from the flowers, leaves, wood, pericarp, etc. of plants; and fragrances from animals such as the musk ox and civet. Examples of synthetic fragrances include hydrocarbons such as monoterpenes, alcohols such as aliphatic alcohols and aromatic alcohols, aldehydes such as terpene aldehydes and aromatic aldehydes, ketones such as alicyclic ketones, esters such as terpene esters, lactones, phenols, oxides, nitrogen-containing compounds and acetals.

### · Antioxidants

The antioxidants are not particularly limited. Examples include carotenoids, ascorbyl stearate, tocopheryl acetate, tocopherol, p-t-butylphenol, butylhydroxyanisole, dibutylhydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin and quercetin.

### · Chelating Agents

Examples of chelating agents include alanine, sodium edetate, sodium polyphosphate, sodium metaphosphate and phosphoric acid.

### · Algefacients

Examples of algefacients include L-menthol, camphor and menthyl lactate.

### · Vitamins

Examples of vitamins include vitamin A compounds such as vitamin A oil, retinol, retinyl acetate and retinyl palmitate; vitamin D compounds such as ergocalciferol and cholecalciferol; vitamin E compounds such as α-tocopherol, β-tocopherol, γ-tocopherol, dl-α-tocopheryl acetate, dl-α-tocopheryl nicotinate and dl-α-tocopheryl succinate; vitamin H and vitamin P.

### · Amino Acids

Examples of amino acids include glycine, valine, leucine, isoleucine, methionine and tryptophan.

### · Nucleic Acids

An example of a nucleic acid is deoxyribonucleic acid.

### · Hormones

Examples of hormones include estradiol and ethinylestradiol.

### · Inclusion Compounds

An example of an inclusion compound is cyclodextrin.

### <Cosmetic>

Emulsified forms of the cosmetic in this invention may be either water-in-oil (W/O) emulsions or oil-in-water-in-oil (O/W/O) emulsions. When a water-breaking cosmetic is applied to the skin, a feeling on use of the inner aqueous phase bursting out is obtained. "Water breaking" refers to the phenomenon of an water-in-oil emulsion breaking down due to shear forces when the cosmetic is applied and the aqueous phase that is the internal phase bursting out as water droplets. Such a feeling on use is obtained by stabilizing a cosmetic in which, under microscopic observation, the emulsified particles are large-size particles or nonuniform in size. The size of these emulsified particles is preferably from 1 to 30 µm, with a size of from 1 to 20 µm being most often observed.

The inventive cosmetic can be prepared by various methods. For example, a powder may be mixed with the oil phase prior to emulsification, mixed with the emulsion following emulsification, or both; the powder is preferably dispersed beforehand in an oil. Preparation may be carried out at room temperature or may be carried out under heating. The mixing method is not particularly limited and may involve the use of, for example, a pulsator, propeller mixer, paddle mixer, dispersion mixer, homogenizing mixer, roll mil, Henschel mixer, atomizer, pin mill or planetary centrifugal mixer.

The cosmetic in this invention is not particularly limited so long as it is a makeup cosmetic, and may be employed as various products, including foundation, makeup base, concealer, cheek color, lipstick, gloss, mascara, eyeshadow, eyeliner, body makeup and nail cosmetics. Of these, makeup cosmetics which include a high level of coloring pigment, such as foundation and concealer, and makeup cosmetics which have been imparted with a sunscreening effect, are preferred. The physical form of the inventive cosmetic may be selected from various physical forms, such as creams, solids, pastes, gels, mousses and sticks.

### EXAMPLES

The invention is illustrated more fully below by way of Examples and Comparative Examples, although the invention is not limited by these Examples. In the following Examples, unless noted otherwise, references to "%" in the composition signify percent by weight and references to "ratio" signify a ratio by weight. The term 'content' refers to the amount of an ingredient included in the formulated product.

### [Examples 1 to 4, Comparative Examples 1 to 3]

Water-in-oil makeup bases formulated as shown in Table 1 were produced.

**[Table 1]**

| Composition (%) | | Example | | | | Comparative Example | | |
|---|---|---|---|---|---|---|---|---|
| | | 1 | 2 | 3 | 4 | 1 | 2 | 3 |
| 1 | KSG-270 (*1) | 3.0 | 3.0 | 3.0 | 3.0 | - | - | - |
| 2 | KSG-210 (*2) | - | - | - | - | 3.0 | 3.0 | 3.0 |
| 3 | KF-6038 (*3) | - | - | - | 0.1 | - | - | 0.1 |
| 4 | Ethylhexyl methoxysilicate | 3.5 | 3.5 | 3.5 | 5.0 | 3.5 | 3.5 | 5.0 |
| 5 | KF-96A-6cs (*4) | - | 0.5 | 1.0 | - | - | 6.5 | - |
| 6 | KF-96L-2cs (*5) | 7.5 | 7.0 | 6.5 | 5.9 | 7.5 | 1.0 | 5.9 |
| 7 | KTP-09W (*6) | 1.0 | 1.0 | 1.0 | 1.0 | 1.0 | 1.0 | 1.0 |
| 8 | BG (butylene glycol) | 5.0 | 5.0 | 5.0 | 5.0 | 5.0 | 5.0 | 5.0 |
| 9 | Sodium chloride | 0.5 | 0.5 | 0.5 | 0.5 | 0.5 | 0.5 | 0.5 |
| 10 | Water | 79.5 | 79.5 | 79.5 | 79.5 | 79.5 | 79.5 | 79.5 |
| Total | | 100.0 | 100.0 | 100.0 | 100.0 | 100.0 | 100.0 | 100.0 |
| | Amount of component (A) | 0.60 | 0.60 | 0.60 | 0.60 | 0.75 | 0.75 | 0.75 |
| | Amount of component (B) | 3.5 | 3.5 | 3.5 | 5.0 | 3.5 | 3.5 | 5.0 |
| | Amount of component (C) | 0 | 0.50 | 1.00 | 0 | 2.25 | 8.75 | 2.25 |
| | Amount of component (D) | 85 | 85 | 85 | 85 | 85 | 85 | 85 |
| | Amount of component (E) | 1 | 1 | 1 | 1 | 1 | 1 | 1 |
| | Amount of component (F) | 2.4 | 2.4 | 2.4 | 2.4 | 0 | 0 | 0 |
| | Combined amount of (B) and (E) | 4.5 | 4.5 | 4.5 | 6.0 | 4.5 | 4.5 | 6.0 |
| | (C)/(B) | - | 0.14 | 0.29 | - | 0.64 | 2.50 | 0.45 |

| | | | | | | | | |
|---|---|---|---|---|---|---|---|---|
| (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 75% Dimethicone (INCI) + 25% Dimethicone/PEG-10/15 Crosspolymer (INCI), from Shin-Etsu Chemical Co., Ltd. (*3) Lauryl PEG-9 Polydimethylsiloxyethyl Dimethicone (INCI), from Shin-Etsu Chemical Co., Ltd. (*4) Dimethicone (INCI) having kinematic viscosity at 25°C of 6 mm²/s, from Shin-Etsu Chemical Co., Ltd. (*5) Dimethicone (INCI) having kinematic viscosity at 25°C of 2 mm²/s, from Shin-Etsu Chemical Co., Ltd. (*6) Hydrophobized titanium oxide, from Shin-Etsu Chemical Co., Ltd. | | | | | | | | |

### (Production Method)

| | |
|---|---|
| Preparation of Oil Phase: | Ingredient 7 was dispersed in Ingredient 6, and the dispersion was uniformly mixed with Ingredients 1 to 5. |
| Preparation of Aqueous Phase: | Ingredients 8 to 10 were uniformly mixed. |

The aqueous phase was quietly added to the oil phase and phase-transfer and emulsification were carried out, giving a makeup base.

Each of the resulting water-in-oil makeup bases was evaluated according to the criteria shown below for: (1) emulsifiability, (2) feel on use (lack of stickiness), (3) water-breaking sensation and (4) ease of application (ease with which cosmetic spreads, and sensation of cosmetic staying in place). The evaluation results are shown in Table 2.

### [Emulsifiability]

The state of the system when transferring the aqueous phase to the oil phase and the appearance during emulsification were observed, based on which the emulsifiability was rated according to the following criteria.

| | |
|---|---|
| ⊚: | Both phase transfer and emulsification are possible (the norm) |
| ○: | Both phase transfer and emulsification are possible but, compared with the norm, 10 to 20% additional time is required for phase transfer. |
| Δ: | Both phase transfer and emulsification are possible but, compared with the norm, 10 to 20% additional time is required for phase transfer, and a trace amount of water droplets remain on the surface of the emulsion (amount of water droplets is less than 0.1 wt%). |
| ×: | Phase transfer is possible, but at least 0.1 wt% and less than 2 wt% of the aqueous phase is discharged during emulsification. |
| ××: | The aqueous phase does not enter the oil phase, or at least 2 wt% of the aqueous phase is discharged during emulsification. |

Ratings of "△" or better are acceptable.

### [Evaluation of Properties]

The feeling on use (lack of stickiness), water-breaking sensation and ease of application were each evaluated according to the criteria shown below on a scale of 1 to 5 by ten expert panelists. The evaluation results were then rated, based on the average score for the ten panelists, according to the rating criteria shown below. Those cosmetics in which phase transfer and emulsification did not occur could not be properly evaluated, and so "--" was entered in the table.

### [Evaluation Criteria]

| | |
|---|---|
| 5 points: | very good |
| 4 points: | good |
| 3 points: | normal |
| 2 points: | somewhat poor |
| 1 point: | poor |

The average score for each makeup base was rated according to the following criteria.

### [Rating Criteria]

| | |
|---|---|
| ⊚: | Average score was 4.5 points or more |
| ○: | Average score was at least 3.5 points but less than 4.5 points |
| Δ: | Average score was at least 2.5 points but less than 3.5 points |
| ×: | Average score was at least 1.5 points but less than 2.5 points |
| ××: | Average score was less than 1.5 points |

Ratings of "○" or better are acceptable.

**[Table 2]**

| Evaluations | Example | | | | Comparative Example | | |
|---|---|---|---|---|---|---|---|
| | 1 | 2 | 3 | 4 | 1 | 2 | 3 |
| Emulsifiability | ⊚ | ○ | Δ | ⊚ | × | ×× | ×× |
| Feeling on use | ⊚ | ⊚ | ⊚ | ⊚ | - | - | - |
| Water-breaking sensation | ⊚ | ⊚ | ⊚ | ⊚ | - | - | - |
| Ease of application | ⊚ | ⊚ | ⊚ | ⊚ | - | - | - |

As is apparent from Table 2, the water-in-oil makeup bases in Examples 1 to 4 were confirmed to have a good feeling on use, good water-breaking sensation and good ease of application. In Comparative Examples 1 to 3, preparation of the cosmetic was difficult. In addition, it was confirmed that the content of component (C) is important for stabilizing emulsification of the inventive cosmetic.

### [Example 5, Comparative Examples 4 and 5]

**[Table 3]**

| Composition (%) | | Example | Comparative Example | |
|---|---|---|---|---|
| | | 5 | 4 | 5 |
| 1 | KSG-270 (*1) | 4.0 | - | - |
| 2 | KSG-210 (*2) | - | 2.7 | 4.0 |
| 3 | KSG-18A (*3) | 1.0 | 1.0 | 1.0 |
| 4 | KF-6038 (*4) | 0.1 | 0.1 | 0.1 |
| 5 | Ethylhexyl methoxysilicate | 7.5 | 7.5 | 7.5 |
| 6 | KF-96L-2cs (*5) | 5.6 | 6.9 | 5.6 |
| 7 | KF-56A (*6) | 4.8 | 4.8 | 4.8 |
| 8 | KF-6115 (*7) | 0.3 | 0.3 | 0.3 |
| 9 | KTP-09W (*8) | 5.1 | 5.1 | 5.1 |
| 10 | KTP-09Y (*9) | 0.58 | 0.58 | 0.58 |
| 11 | KTP-09R (*9) | 0.25 | 0.25 | 0.25 |
| 12 | KTP-09B (*9) | 0.07 | 0.07 | 0.07 |
| 13 | Hydrophobized finely divided zinc oxide (*10) | 6.0 | 6.0 | 6.0 |
| 14 | BG (butylene glycol) | 8.0 | 8.0 | 8.0 |
| 15 | Sodium citrate | 0.2 | 0.2 | 0.2 |
| 16 | Magnesium sulfate | 0.5 | 0.5 | 0.5 |
| 17 | Water | 56.0 | 56.0 | 56.0 |
| Total | | 100.00 | 100.00 | 100.00 |
| | Amount of component (A) | 0.8 | 0.7 | 1.0 |
| | Amount of component (B) | 7.5 | 7.5 | 7.5 |
| | Amount of component (C) | 0 | 2.03 | 3.0 |
| | Amount of component (D) | 64.7 | 64.7 | 64.7 |
| | Amount of component (E) | 12.0 | 12.0 | 12.0 |
| | Amount of component (F) | 3.2 | 0 | 0 |
| | Combined amount of (B) and (E) | 19.5 | 19.5 | 19.5 |
| | (C)/(B) | - | 0.27 | 0.40 |

| | | | | |
|---|---|---|---|---|
| (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 75% Dimethicone (INCI) having kinematic viscosity at 25°C of 6 mm²/s + 25% Dimethicone/PEG-10/15 Crosspolymer (INCI), from Shin-Etsu Chemical Co., Ltd. (*3) A mixture of 85% Diphenylsiloxy Phenyl Trimethicone (INCI) + 15% Dimethicone/Phenyl Vinyl Dimethicone Crosspolymer (INCI), from Shin-Etsu Chemical Co., Ltd. (*4) Lauryl PEG-9 Polydimethylsiloxyethyl Dimethicone (INCI), from Shin-Etsu Chemical Co., Ltd. (*5) Dimethicone (INCI) having kinematic viscosity at 25°C of 2 mm²/s, from Shin-Etsu Chemical Co., Ltd. (*6) Diphenylsiloxy Phenyl Trimethicone (INCI), from Shin-Etsu Chemical Co., Ltd. (*7) Lauryl Polyglyceryl-3-Polydimethylsiloxyethyl Dimethicone (INCI), from Shin-Etsu Chemical Co., Ltd. (*8) Hydrophobized Titanium Oxide, from Shin-Etsu Chemical Co., Ltd. (*9) Hydrophobized Iron Oxide, from Shin-Etsu Chemical Co., Ltd. (*10) Treated with AES-3083, from Shin-Etsu Chemical Co., Ltd. | | | | |

### (Production Method)

| | |
|---|---|
| Preparation of Oil Phase: | Ingredients 1 to 6 were uniformly mixed. |
| Preparation of Aqueous Phase: | Ingredients 14 to 17 were uniformly mixed. |
| Preparation of Paste Phase: | Ingredients 9 to 13 were mixed into Ingredients 7 and 8, and dispersed on a three-roll mill. |

The aqueous phase was quietly added to the oil phase and phase-transfer and emulsification were carried out, following which the paste was mixed in, thereby giving a foundation. The same evaluations as described above were carried out on the resulting foundations. The results are presented in the table.

**[Table 4]**

| Evaluations | Example | Comparative Example | |
|---|---|---|---|
| | 5 | 4 | 5 |
| Emulsifiability | ⊚ | ×× | ×× |
| Feeling on use | ○ | -- | -- |
| Water-breaking sensation | ⊚ | -- | -- |
| Ease of application | ⊚ | -- | -- |

As is apparent from Table 4, the water-in-oil foundation in Example 5 was confirmed to have a good feeling on use, good water-breaking sensation and good ease of application. In Comparative Examples 4 and 5, preparation as a cosmetic was difficult.

### [Example 6]

### Water-in-Oil Concealer

### <Preparation of Cosmetic>

A: Ingredients 8 to 13 were dispersed on a three-roll mill.
B: Ingredients 1 to 7 were uniformly mixed.
C: Ingredients 16 to 21 were uniformly mixed.
D: C was added to B and emulsified, following which A and Ingredients 14 and 15 were added and mixed, giving a water-in-oil concealer.

| Ingredients | | Weight (%) | |
|---|---|---|---|
| 1. | KSG-270 (*1) | | 5 |
| 2. | KSG-18A (*2) | | 1 |
| 3. | KF-6038 (*3) | | 0.5 |
| 4. | Homosalate | | 3.5 |
| 5. | C13-15 Alkane | | 6.5 |
| 6. | Cetyl ethylhexanoate | | 3 |
| 7. | Caprylic/capric triglyceride | | 2 |
| 8. | Isotridecyl isononanoate | | 3 |
| 9. | KP-578 (*4) | | 0.5 |
| 10. | KTP-09W (*5) | | 13 |
| 11. | KTP-09R (*5) | | 0.7 |
| 12. | KTP-09Y (*5) | | 1.2 |
| 13. | KTP-09B (*5) | | 0.1 |
| 14. | KSP-300 (*6) | | 6 |
| 15. | KMP-590 (*7) | | 4 |
| 16. | Sorbitol | | 3 |
| 17. | Pentylene glycol | | 2 |
| 18. | BG | | 5 |
| 19. | Sodium citrate | | 0.2 |
| 20. | Magnesium sulfate | | 1 |
| 21. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 85% Diphenylsiloxy Phenyl Trimethicone (INCI) + 15% Dimethicone/Phenyl Vinyl Dimethicone Crosspolymer (INCI), from Shin-Etsu Chemical Co., Ltd. (*3) Lauryl PEG-9 Polydimethylsiloxyethyl Dimethicone (INCI), from Shin-Etsu Chemical Co., Ltd. (*4) Acrylates/Ethylhexyl Acrylate/Dimethicone Methacrylate Copolymer) (INCI), from Shin-Etsu Chemical Co., Ltd. (*5) KF-9909-treated inorganic coloring pigments: W = white, R = red, Y = yellow, B = black (*6) Diphenyl Dimethicone/Vinyl Diphenyl Dimethicone/Silsesquioxane Crosspolymer (INCI), from Shin-Etsu Chemical Co., Ltd. (*7) Polymethylsilsesquioxane (INCI), from Shin-Etsu Chemical Co., Ltd. | | | |

The resulting water-in-oil concealer had a good feel on use, good water-breaking sensation and good ease of application.

### [Example 7]

### Water-in-Oil Cheek

### <Preparation of Cosmetic>

A: Ingredients 7 to 14 were dispersed on a three-roll mill.
B: Ingredients 1 to 6 were uniformly mixed.
C: Ingredients 16 to 21 were uniformly mixed.
D: C was added to B and emulsified, following which A and Ingredient 15 were added, giving a water-in-oil cheek.

| Ingredients | | Weight (%) | |
|---|---|---|---|
| 1. | KSG-270 (*1) | | 5 |
| 2. | KSG-18A (*2) | | 2 |
| 3. | KF-6038 (*3) | | 0.1 |
| 4. | Ethylhexyl methoxycinnamate | | 5 |
| 5. | KF-4422 (*4) | | 2 |
| 6. | KF-96L-2cs (*5) | | 8 |
| 7. | Mica | | 0.3 |
| 8. | Red 202 | | 0.1 |
| 9. | Yellow 4 | | 0.3 |
| 10. | KP-574-treated titanium oxide (*6) | | 0.8 |
| 11. | KP-574-treated black iron oxide (*6) | | 0.06 |
| 12. | Red 201 | | 0.04 |
| 13. | KP-574-treated red iron oxide (*6) | | 0.2 |
| 14. | Polyglyceryl-2 triisostearate | | 1.2 |
| 15. | KSP-441 (*7) | | 1.5 |
| 16. | Glycerin | | 3 |
| 17. | 1,2-Hexanediol | | 2 |
| 18. | DPG | | 8 |
| 19. | Sodium citrate | | 0.2 |
| 20. | Sodium chloride | | 1 |
| 21. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 85% Diphenylsiloxy Phenyl Trimethicone (INCI) + 15% Dimethicone/Phenyl Vinyl Dimethicone Crosspolymer (INCI), from Shin-Etsu Chemical Co., Ltd. (*3) Lauryl PEG-9 Polydimethylsiloxyethyl Dimethicone (INCI), from Shin-Etsu Chemical Co., Ltd. (*4) Ethyl Methicone (INCI), from Shin-Etsu Chemical Co., Ltd. (*5) Dimethicone (INCI) having kinematic viscosity at 25°C of 2 mm²/s, from Shin-Etsu Chemical Co., Ltd. (*6) Acrylates/Tridecyl Acrylate/Triethoxysilylpropyl Methacrylate/Dimethicone Methacrylate Copolymer (INCI), from Shin-Etsu Chemical Co., Ltd. (*7) Vinyl Dimethicone/Methicone Silsesquioxane Crosspolymer (INCI), from Shin-Etsu Chemical Co., Ltd. | | | |

The resulting water-in-oil cheek had a good feel on use, good water-breaking sensation and good ease of application.

### [Example 8]

### Water-in-Oil Cast Foundation

### <Preparation of Cosmetic>

A: Ingredients 11 to 16 were dispersed on a three-roll mill.
B: Ingredients 1 to 10 were uniformly mixed at 90°C.
C: Ingredients 17 to 22 were uniformly mixed.
D: C was added to B and emulsified, then filled into a predetermined container, giving a water-in-oil cast foundation.

| Ingredients | | Weight (%) | |
|---|---|---|---|
| 1. | KSG-270 (*1) | | 3 |
| 2. | KSG-042Z (*2) | | 1 |
| 3. | KF-6048 (*3) | | 0.3 |
| 4. | Ethylhexyl methoxycinnamate | | 7 |
| 5. | Octocrylene | | 5 |
| 6. | Diethylamino hydroxybenzoyl hexyl benzoate | | 3 |
| 7. | Ethylhexyl salicylate | | 5 |
| 8. | KF-4422 (*4) | | 3 |
| 9. | Hexyl laurate | | 8 |
| 10. | Ceresin | | 5 |
| 11. | Ethylhexyl palmitate | | 2 |
| 12. | Alkylsilane-treated titanium oxide (*5) | | 5 |
| 13. | Alkylsilane-treated red iron oxide (*5) | | 0.2 |
| 14. | Alkylsilane-treated yellow iron oxide (*5) | | 0.7 |
| 15. | Alkylsilane-treated black iron oxide (*5) | | 0.1 |
| 16. | KF-6115 (*6) | | 0.2 |
| 17. | Diglycerin | | 1 |
| 18. | Ethylhexylglycerin | | 0.1 |
| 19. | BG | | 8 |
| 20. | Sodium citrate | | 0.2 |
| 21. | Sodium chloride | | 1 |
| 22. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 20% Lauryl Polydimethylsiloxyethyl Dimethicone/Bis-Vinyl Dimethicone Crosspolymer and 80% Isododecane (INCI), from Shin-Etsu Chemical Co., Ltd. (*3) Cetyl PEG/PPG-10/1 Dimethicone (INCI), from Shin-Etsu Chemical Co., Ltd. (*4) Ethyl Methicone (INCI), from Shin-Etsu Chemical Co., Ltd. (*5) AES-3083-treated inorganic coloring pigments: W = white, R = red, Y = yellow, B = black (*6) Lauryl Polyglyceryl-3-Polydimethylsiloxyethyl Dimethicone (INCI), from Shin-Etsu Chemical Co., Ltd. | | | |

The resulting water-in-oil cast foundation had a good feel on use, good water-breaking sensation and good ease of application.

### [Example 9]

### Water-in-Oil Stick Foundation

### <Preparation of Cosmetic>

A: Ingredients 11 to 16 were dispersed on a three-roll mill.
B: Ingredients 1 to 10 were uniformly mixed at 90°C.
C: Ingredients 17 to 22 were uniformly mixed at 90°C.
D: C was added to B and emulsified, then filled into a predetermined container, giving a water-in-oil stick foundation.

| Ingredients | | Weight (%) | |
|---|---|---|---|
| 1. | KSG-270 (*1) | | 3 |
| 2. | KSG-18A (*2) | | 1 |
| 3. | KF-6048 (*3) | | 0.5 |
| 4. | Ethylhexyl salicylate | | 3 |
| 5. | Bis-ethylhexyloxyphenol methoxyphenyl triazine | | 2 |
| 6. | Caprylic/capric triglyceride | | 5 |
| 7. | KF-4422 (*4) | | 2 |
| 8. | KF-96L-1.5cs | | 5 |
| 9. | Synthetic wax | | 6 |
| 10. | Microcrystalline wax | | 2 |
| 11. | Cetyl ethylhexanoate | | 5 |
| 12. | KTP-09W (*5) | | 13 |
| 13. | KTP-09R (*5) | | 0.7 |
| 14. | KTP-09Y (*5) | | 1.2 |
| 15. | KTP-09B (*5) | | 0.1 |
| 16. | KF-6115 (*6) | | 0.5 |
| 17. | Betaine | | 1 |
| 18. | Methylparaben | | 0.1 |
| 19. | BG | | 8 |
| 20. | Sodium citrate | | 0.2 |
| 21. | Sodium chloride | | 1 |
| 22. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 85% Diphenylsiloxy Phenyl Trimethicone (INCI) + 15% Dimethicone/Phenyl Vinyl Dimethicone Crosspolymer (INCI), from Shin-Etsu Chemical Co., Ltd. (*3) Cetyl PEG/PPG-10/1 Dimethicone (INCI), from Shin-Etsu Chemical Co., Ltd. (*4) Ethyl Methicone (INCI), from Shin-Etsu Chemical Co., Ltd. (*5) KF-9909-treated inorganic coloring pigments: W = white, R = red, Y = yellow, B = black (*6) Lauryl Polyglyceryl-3-Polydimethylsiloxyethyl Dimethicone (INCI), from Shin-Etsu Chemical Co., Ltd. | | | |

The resulting water-in-oil stick foundation had a good feel on use, good water-breaking sensation and good ease of application.

### [Example 10]

### Water-in-Oil Foundation

### <Preparation of Cosmetic>

A: Ingredients 7 to 12 were dispersed on a three-roll mill.
B: Ingredients 1 to 6 were uniformly mixed.
C: Ingredients 14 to 19 were uniformly mixed.
D: C was added to B and emulsified, following which A and Ingredient 13 were added, giving a water-in-oil foundation.

| Ingredients | | Weight (%) | |
|---|---|---|---|
| 1. | KSG-270 (*1) | | 5 |
| 2. | KSG-18A (*2) | | 2 |
| 3. | KF-6038 (*3) | | 0.3 |
| 4. | Ethylhexyl methoxycinnamate | | 5 |
| 5. | Diethylamino hydroxybenzoyl hexyl benzoate | | 2.5 |
| 6. | TMF-1.5 (*4) | | 9 |
| 7. | Isononyl isononanoate | | 2 |
| 8. | KF-9901-treated titanium oxide (*5) | | 8.5 |
| 9. | KF-9901-treated red iron oxide (*5) | | 0.4 |
| 10. | KF-9901-treated yellow iron oxide (*5) | | 1 |
| 11. | KF-9901-treated black iron oxide (*5) | | 0.1 |
| 12. | KP-578 (*6) | | 0.3 |
| 13. | KSP-105 (*7) | | 2 |
| 14. | Glycerin | | 3 |
| 15. | Phenoxyethanol | | 0.3 |
| 16. | BG | | 5 |
| 17. | Sodium citrate | | 0.2 |
| 18. | Sodium chloride | | 1 |
| 19. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 85% Diphenylsiloxy Phenyl Trimethicone (INCI) + 15% Dimethicone/Phenyl Vinyl Dimethicone Crosspolymer (INCI), from Shin-Etsu Chemical Co., Ltd. (*3) Lauryl PEG-9 Polydimethylsiloxyethyl Dimethicone (INCI), from Shin-Etsu Chemical Co., Ltd. (*4) Methyl Trimethicone (INCI), from Shin-Etsu Chemical Co., Ltd. (*5) Hydrogen Dimethicone (INCI), from Shin-Etsu Chemical Co., Ltd. (*6) Acrylates/Ethylhexyl Acrylate/Dimethicone Methacrylate Copolymer (INCI), from Shin-Etsu Chemical Co., Ltd. (*7) Vinyl Dimethicone/Methicone Silsesquioxane Crosspolymer (INCI), from Shin-Etsu Chemical Co., Ltd. | | | |

The resulting water-in-oil foundation had a good feel on use, good water-breaking sensation and good ease of application.

### [Example 11]

### Water-in-Oil Foundation

### <Preparation of Cosmetic>

A: Ingredients 8 to 15 were dispersed on a three-roll mill.
B: Ingredients 1 to 7 were uniformly mixed.
C: Ingredients 16 to 21 were uniformly mixed.
D: C was added to B and emulsified, giving a water-in-oil foundation.

| Ingredients | | Weight (%) | |
|---|---|---|---|
| 1. | KSG-270 (*1) | | 5 |
| 2. | KSG-42A (*2) | | 1 |
| 3. | KF-6048 (*3) | | 0.3 |
| 4. | Octocrylene | | 5 |
| 5. | Diethylamino hydroxybenzoyl hexyl benzoate | | 2 |
| 6. | Ethylhexyl salicylate | | 5 |
| 7. | KF-4422 (*4) | | 8 |
| 8. | Ethylhexyl palmitate | | 5 |
| 9. | KTP-09W (*5) | | 5 |
| 10. | KTP-09R (*5) | | 0.2 |
| 11. | KTP-09Y (*5) | | 0.7 |
| 12. | KTP-09B (*5) | | 0.1 |
| 13. | KF-6115 (*6) | | 0.5 |
| 14. | Metal soap-treated finely divided titanium oxide | | 2 |
| 15. | Silicone-treated finely divided zinc oxide | | 4 |
| 16. | Glycerin | | 2 |
| 17. | Ethanol | | 5 |
| 18. | BG | | 5 |
| 19. | Sodium citrate | | 0.2 |
| 20. | Magnesium sulfate | | 1 |
| 21. | Water | balance | |
| | Total | 100 | |

| | | | |
|---|---|---|---|
| Amount of Ingredient (C): 0% (* 1) A mixture of 80% Diphenylsiloxy Phenyl Trimethicone (INCI) + 20% Dimethicone/PEG-10/15 Crosspolymer (INCI), from Shin-Etsu Chemical Co., Ltd. (*2) A mixture of 20% Vinyl Dimethicone/Lauryl Dimethicone Crosspolymer and 80% Isododecane (INCI), from Shin-Etsu Chemical Co., Ltd. (*3) Cetyl PEG/PPG-10/1 Dimethicone (INCI), from Shin-Etsu Chemical Co., Ltd. (*4) Ethyl Methicone (INCI), from Shin-Etsu Chemical Co., Ltd. (*5) KF-9909-treated inorganic coloring pigments: W = white, R = red, Y = yellow, B = black (*6) Lauryl Polyglyceryl-3-Polydimethylsiloxyethyl Dimethicone (INCI), from Shin-Etsu Chemical Co., Ltd. | | | |

The resulting water-in-oil foundation had a good feel on use, good water-breaking sensation and good ease of application.

## Claims

1. A water-breaking makeup cosmetic comprising:
(A) from 0.1 to 4 wt% of a crosslinked polyether-modified silicone having a silicone chain crosslinked with polyether chains,
(B) from 3.5 to 20 wt% of an ultraviolet absorber,
(C) 1.9 wt% or less of one or more compound selected from cyclic or linear dimethylpolysiloxanes having a kinematic viscosity at 25°C of from 4 to 100 mm²/s,
(D) from 40 to 90 wt% of an aqueous ingredient, and
(E) from 1 to 25 wt% of a powder.

2. The water-breaking makeup cosmetic of claim 1, wherein the combined amount of components (B) and (E) is from 4.5 to 45 wt% of the overall cosmetic.

3. The water-breaking makeup cosmetic of claim 1 or 2, wherein component (A) is dimethicone/PEG-10/15 crosspolymer.

4. The water-breaking makeup cosmetic of any one of claims 1 to 3, comprising as component (D) from 38 to 85 wt% of water, based on the overall cosmetic.

5. The water-breaking makeup cosmetic of any one of claims 1 to 4, further comprising (F) from 0.1 to 20 wt% of diphenylsiloxy phenyl trimethicone.

6. The water-breaking makeup cosmetic of any one of claims 1 to 5, further comprising (G) from 0.1 to 20 wt% of ethyl methicone.

7. The water-breaking makeup cosmetic of any one of claims 1 to 6, further comprising (H) from 0.1 to 1.0 wt% of a non-crosslinked silicone surfactant.
